(19) Europäisches Patentamt
European Patent Office
Office européen des brevets

(11) Veröffentlichungsnummer : **0 009 566**
**B1**

(12) # EUROPÄISCHE PATENTSCHRIFT

(45) Veröffentlichungstag der Patentschrift : **09.09.81**

(51) Int. Cl.³ : **C 07 F 9/65**, C 07 D239/34, A 01 N 57/10

(21) Anmeldenummer : **79102795.6**

(22) Anmeldetag : **03.08.79**

(54) **2-Cycloalkyl-pyrimidin(5)yl-(thiono)(thiol)-phosphor-(phosphon)-säureester bzw. -esteramide, Verfahren zu ihrer Herstellung sowie ihre Verwendung als Insektizide, Akarizide und Nematizide.**

(30) Priorität : **12.08.78 DE 2835492**

(43) Veröffentlichungstag der Anmeldung : **16.04.80 (Patentblatt 80/08)**

(45) Bekanntmachung des Hinweises auf die Patenterteilung : **09.09.81 Patentblatt 81/36**

(84) Benannte Vertragsstaaten : **AT BE CH DE FR GB IT NL SE**

(56) Entgegenhaltungen :
**FR - A - 2 365 577**
**US - A - 4 012 506**

(73) Patentinhaber : **BAYER AG**
**Zentralbereich Patente, Marken und Lizenzen**
**D-5090 Leverkusen 1, Bayerwerk (DE)**

(72) Erfinder : **Maurer, Fritz, Dr.**
**Roeberstrasse 8**
**D-5600 Wuppertal 1 (DE)**
Erfinder : **Homeyer, Bernhard, Dr.**
**Obere Strasse 28**
**D-5090 Leverkusen 3 (DE)**
Erfinder : **Hammann, Ingeborg, Dr.**
**Belfortstrasse 9**
**D-5000 Koeln 1 (DE)**
Erfinder : **Behrenz, Wolfgang, Dr.**
**Untergruendemich 14**
**D-5063 Overath (DE)**

**EP 0 009 566 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

2-Cycloalkyl-pyrimidin(5)yl-(thiono) (thiol)-phosphor-(phosphon)-säureester bzw. -esteramide, Verfahren zu ihrer Herstellung sowie ihre Verwendung als Insektizide, Akarizide und Nematizide

Die Erfindung betrifft neue 2-Cycloalkyl-pyrimidin(5)yl-(thiono) (thiol)-phosphor (phosphon)-säureester bzw. -esteramide, ein Verfahren zu ihrer Herstellung sowie ihre Verwendung als Insektizide, Akarizide und Nematizide.

Es ist bekannt, daß bestimmte 2,6-Dialkyl-pyrimidin(4)-yl-thiono (thiol)-phosphorsäureester, wie z.B. O,O-Diäthyl-O-(2-iso-propyl-6-methyl-pyrimidin(4)yl)-thiono-phosphorsäureester und O-Äthyl-S-n-propyl-O-(2-iso-propyl-6-methyl-pyrimidin(4)yl)-thionothiolphosphorsäureester, insektizid und akarizid wirksam sind (vergleiche DE-PS 910 652 und DE-OS 2 360 877). Weiterhin sind aus der FR-A-2 365 577 bestimmte 2-Alkyl-pyrimidin(5)yl-(thiono) (thiol)-phosphor (phosphon)-säureester bzw. -esteramide und ihre Verwendung als Insektizide, Akarizide und Nematizide und aus der US-A-4 012 506 bestimmte 2-Cycloalkyl-4-methylpyrimidin(6)yl-thiono-phosphorsäureester und ihre Verwendung als Insektizide und Akarizide bekannt geworden.

Die Wirkung dieser Verbindungen ist jedoch, insbesondere bei niedrigen Aufwandmengen und Konzentrationen, nicht immer zufriedenstellend.

Es wurden nun neue 2-Cycloalkyl-pyrimidin(5)yl-(thiono) (thiol)-phosphor (phosphon)-säureester bzw. -esteramide der Formel (I) gefunden,

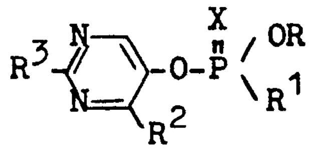

(I)

in welcher

R für Alkyl steht,

$R^1$ für Alkyl, Alkoxy, Alkylthio, Alkylamino oder Phenyl steht,

$R^2$ für Wasserstoff oder Alkyl steht,

$R^3$ für Cycloalkyl steht und

X für Sauerstoff oder Schwefel steht.

Man erhält die neuen 2-Cycloalkylpyrimidin(5)yl-(thiono) (thiol)-phosphor (phosphon)-säureester bzw. -esteramide der Formel (I), wenn man (Thiono) (Thiol) Phosphor (phosphon)-säureesterhalogenide bzw. (Thiono)-phosphorsäureester-amidhalogenide der Formel II

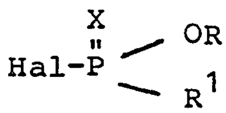

(II)

in welcher

R, $R^1$ und X die oben angegebene Bedeutung haben und

Hal für Chlor oder Brom steht, mit 5-Hydroxy-pyrimidinen der Formel III

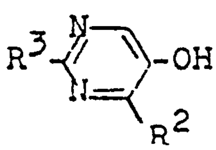

(III)

in welcher

$R^2$ und $R^3$ die oben angegebene Bedeutung haben, gegebenenfalls in Gegenwart eines Säureakzeptors und gegebenenfalls in Gegenwart eines inerten Verdünnungsmittels umsetzt.

Es wurden ferner die neuen 5-Hydroxy-pyrimidine der Formel III gefunden

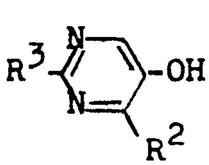

in welcher

$R^2$ und $R^3$ die oben angegebene Bedeutung haben.

Man erhält die 5-Hydroxypyrimidine der Formel III, wenn man Amidin-hydrochloride der Formel IV

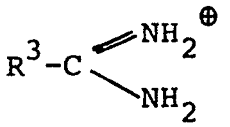

(IV)

in welcher

$R^3$ die oben angegebene Bedeutung hat, mit Acroleinderivaten der Formel V

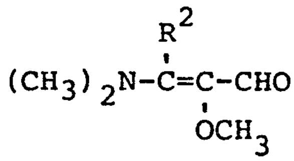

(V)

in welcher

$R^2$ die oben angegebene Bedeutung hat, in Gegenwart einer Base und gegebenenfalls in Gegenwart eines Verdünnungsmittels bei Temperaturen zwischen 0 und 100 °C umsetzt und in der Folge nach Zugabe überschüssiger wäßriger Kalilauge mehrere Stunden im Autoklaven auf 180 bis 200 °C erhitzt.

Die neuen Verbindungen der Formel (I) zeichnen sich durch starke insektizide, akarizide und nematizide Wirksamkeit aus.

Überraschenderweise zeigen die erfindungsgemäßen 2-Cycloalkyl-pyrimidin(5)yl-(thiono) (thiol)-phosphor (phosphon)-säureester bzw. -esteramide eine bessere insektizide, akarizide und nematizide Wirkung als die entsprechenden aus dem Stand der Technik bekannten Verbindungen analoger Konstitution und gleicher Wirkungsrichtung.

Von den erfindungsgemäßen Verbindungen der Formel I sind bevorzugt diejenigen in denen R, $R^1$, $R^2$, $R^3$ und X die bei den Ausgangsprodukten der Formeln II und III angegebenen bevorzugten Definitionen besitzen.

Verwendet man beispielsweise 2-Cyclobutyl-5-hydroxypyrimidin und O-Äthyl-äthanphosphonsäureesterchlorid als Ausgangsstoffe, so kann die Reaktion durch folgendes Formelschema skizziert werden :

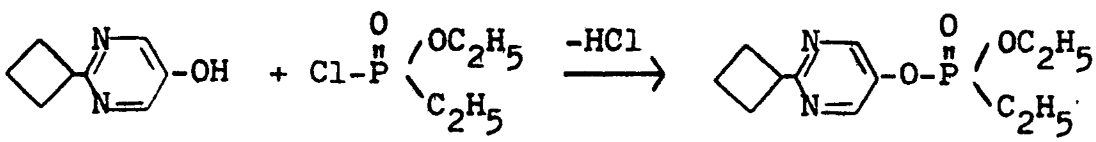


Die als Ausgangsstoffe zu verwendenden (Thiono) (Thiol)-Phosphor (phosphon)-säureesterhalogenide bzw. (Thiono)-phosphorsäureesteramidhalogenide sind durch Formel (II) definiert.

Vorzugsweise stehen darin

R für geradkettiges oder verzweigtes Alkyl mit 1 bis 5, insbesondere 1 bis 3 Kohlenstoffatomen,

$R^1$ für geradkettiges oder verzweigtes Alkyl mit 1 bis 5, insbesondere 1 bis 3 Kohlenstoffatomen, geradkettiges oder verzweigtes Alkoxy, Alkylthio oder Alkylamino mit jeweils 1 bis 5, insbesondere 1 bis 3 Kohlenstoffatomen je Alkylrest oder für Phenyl,

X für Sauerstoff oder Schwefel und

Hal für Chlor.

Die (Thiono) (Thiol)-phosphor (phosphon)-säureester-halogenide bzw. (Thiono)-phosphorsäureesteramidhalogenide der Formel (II) sind bekannte Verbindungen. Als Beispiele seien genannt :

O-Methyl-, O-Äthyl-, O-n-Propyl-, O-iso-Propyl-, -methan-, -äthan-, -propan- bzw. -phenylphosphonsäureesterchlorid und die entsprechenden Thionoanalogen, ferner

O,O-Dimethyl-, O,O-Diäthyl-, O,O-Di-n-propyl-, O-Methyl-O-äthyl-, O-Methyl-O-n-propyl-, O-Methyl-O-iso-propyl-, O-Äthyl-O-n-propyl- und O-Äthyl-O-iso-propyl-phosphorsäurediesterchlorid und die entsprechenden Thionoanalogen, ferner

O,S-Dimethyl-, O,S-Diäthyl-, O,S-Di-n-propyl-, O,S-Diiso-propyl-, O-Äthyl-S-n-propyl-, O-Äthyl-S-iso-propyl-, O-Äthyl-S-sec.-butyl-, O-n-Propyl-S-äthyl-, O-n-Propyl-S-iso-propyl- und O-iso-Propyl-S-n-propylthiolphosphorsäurediesterchlorid und die entsprechenden Thionoanalogen, ferner

O-Methyl-N-methyl-, O-Methyl-N-äthyl-, O-Methyl-N-propyl-, O-Methyl-N-iso-propyl, O-Äthyl-N-methyl-, O-Äthyl-N-athyl-, O-Äthyl-N-n-propyl-, O-Äthyl-N-iso-propyl-, O-n-Propyl-N-methyl-, O-n-Propyl-N-äthyl-, O-n-Propyl-N-n-propyl-, O-n-Propyl-N-iso-propyl-, O-iso-Propyl-N-methyl-, O-iso-Propyl-N-äthyl-, O-iso-Propyl-N-n-propyl- und O-iso-Propyl-N-iso-propyl-phosphorsäureesteramidchlorid und die entsprechenden Thionoanalogen.

Die weiter als Ausgangsstoffe zu verwendenden 5-Hydroxypyrimidine sind durch Formel (III) definiert.

Vorzugsweise stehen darin

$R^2$ für Wasserstoff oder Methyl und

$R^3$ für Cycloalkyl mit 3 bis 6 Kohlenstoffatomen.

Als Beispiele seien im einzelnen genannt:

2-Cyclopropyl-5-hydroxy-pyrimidin,
2-Cyclobutyl-5-hydroxy-pyrimidin,
2-Cyclopentyl-5-hydroxy-pyrimidin,
2-Cyclohexyl-5-hydroxy-pyrimidin,
2-Cyclopropyl-4-methyl-5-hydroxy-pyrimidin,
2-Cyclobutyl-4-methyl-5-hydroxy-pyrimidin,
2-Cyclopentyl-4-methyl-5-hydroxy-pyrimidin und
2-Cyclohexyl-4-methyl-5-hydroxy-pyrimidin.

Die 5-Hydroxy-pyrimidine der Formel (III) sind neue Verbindungen. Man erhält sie, indem man Amidin-hydrochloride der Formel IV

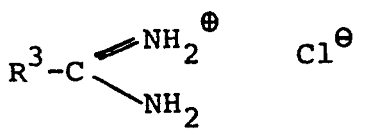

(IV)

in welcher

$R^3$ die oben angegebene Bedeutung hat, mit Acroleinderivaten der Formel V

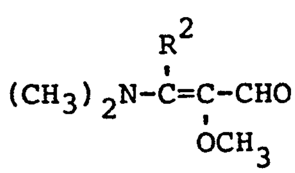

(V)

in welcher

$R^2$ die oben angegebene Bedeutung hat, in Gegenwart einer Base wie z.B. Natriummethylat und gegebenenfalls in Gegenwart eines Verdünnungsmittels wie z.B. Methanol bei Temperaturen zwischen 0 und 100 °C umsetzt und in der Folge nach Zugabe überschüssiger wässriger Kalilauge mehrere Stunden im Autoklaven auf 180 bis 200 °C erhitzt. Zur Aufarbeitung wird eingeengt, mit wenig Wasser verdünnt und ein pH-Wert zwischen 4 und 5 eingestellt. Die Produkte der Formel (III) fallen hierbei kristallin an.

Amidin-hydrochloride der Formel (IV) sind bekannte Verbindungen (vergleiche US-PS 4 012 506). Als Beispiele seien genannt:

Cyclopropylamidin-hydrochlorid,
Cyclobutylamidin-hydrochlorid,
Cyclopentylamidin-hydrochlorid und
Cyclohexylamidin-hydrochlorid.

Acroleinderivate der Formel (V) sind ebenfalls bekannt (vergleiche Archiv der Pharmazie *300* (1967), 704-708).

Als Beispiele hierfür seien genannt:

2-Methoxy-3-dimethylamino-acrolein und
2-Methoxy-3-dimethylamino-3-methyl-acrolein.

Das Verfahren zur Herstellung der erfindungsgemäßen Cycloalkyl-pyrimidin(5)yl-(thiono) (thiol)-phosphor-(phosphon)-säureester bzw. -esteramide wird bevorzugt unter Verwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Als Säureakzeptoren können alle üblichen Saürebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Die Reaktionstemperatur kann innerhalb eines größeren Bereichs variiert werden. Im allgemeinen arbeitet man zwischen 0 und 80 °C, vorzugsweise bei 20 bis 60 °C. Das erfindungsgemäße Verfahren wird im allgemeinen bei Normaldruck durchgeführt.

Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt

keine wesentlichen Vorteile. Die Umsetzung wird im allgemeinen in einem geeigneten Verdünnungsmittel in Gegenwart eines Säureakzeptors durchgeführt und das Reaktionsgemisch wird mehrere Stunden bei der erforderlichen Temperatur gerührt. Danach gibt man eine organisches Lösungsmittel, z.B. Toluol, zu und arbeitet die organische Phase wie üblich durch Waschen, Trocknen und Abdestillieren des Lösungsmittels auf.

Die neuen Verbindungen fallen in Form von Ölen an, die sich zum Teil nicht unzersetzt destillieren lassen, jedoch durch sogenanntes « Andestillieren », d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören :

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Phemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chrorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Die Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphtaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierung Haftmittel wie Carboxy-methylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen 2-Cycloalkyl-pyrimidin(5)yl-(thiono) (thiol)-phosphor (phosphon)-säureester bzw. -esteramide besitzen auch eine gute wurzelsystemische Wirkung gegen saugende und fressende Insekten und Milben.

Beispiel A

Grenzkonzentrations-Test/Bodeninsekten

| | | | |
|---|---|---|---|
| Testinsekt | Phorbia-antiqua-Maden (im Boden) | | |
| Lösungsmittel | 3 | Gewichtsteile | Aceton |
| Emulgator | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge

pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik : 7, 16.

## Beispiel B

Grenzkonzentrations-Test/Bodeninsekten

| | | | |
|---|---|---|---|
| Testinsekt | Tenebrio-molitor-Larven(im Boden) | | |
| Lösungsmittel | 3 | Gewichtsteile | Aceton |
| Emulgator | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik : 1, 4, 7.

## Beispiel C

Grenzkonzentrations-Test/Wurzelsystemische Wirkung

| | | | |
|---|---|---|---|
| Testinsekt | Myzus persicae | | |
| Lösungsmittel | 3 | Gewichtsteile | Aceton |
| Emulgator | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik : 4, 5.

## Beispiel D

Grenzkonzentrations-Test/Wurzelsystemische Wirkung

| | | | |
|---|---|---|---|
| Testinsekt | Phaedon cochleariae-Larven | | |
| Lösungsmittel | 3 | Gewichtsteile | Aceton |
| Emulgator | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdunnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik : 4, 5.

### Beispiel E

Grenzkonzentrations-Test/Nematoden

| | | | |
|---|---|---|---|
| Testnematode | Meloidogyne incognita | | |
| Lösungsmittel | 3 | Gewichtsteile | Aceton |
| Emulgator | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27 °C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele überlegene Wirksamkeit gegenüber dem Stand der Technik : 4, 5.

### Beispiel F

$LD_{100}$-Test

| | |
|---|---|
| Testtiere | Sitophilus granarius |
| Zahl der Testtiere | 25 |
| Lösungsmittel | Aceton |

2 Gewichtsteile Wirkstoff werden in 1 000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden ; 0 % bedeutet, daß keine Testtiere abgetötet wurden.

Bei diesem Test zeigen z.B. die folgenden Verbindungen der Herstellungsbeispiele überlegene Wirkung gegenüber dem Stand der Technik : 4, 6.

### Beispiel G

Laphygma-Test

| | | | |
|---|---|---|---|
| Lösungsmittel | 3 | Gewichtsteile | Aceton |
| Emulgator | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100 %, daß alle Raupen abgetötet wurden ; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik : 13, 4, 1, 7, 16.

### Beispiel H

Tetranychus-Test (resistent)

| | | | |
|---|---|---|---|
| Lösungsmittel | 3 | Gewichtsteile | Aceton |
| Emulgator | 1 | Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden ; 0 % bedeutet, daß keine Spinnmilben abgetötet wurden.

Bei diesem Test zeigen z.B. die folgenden Verbindungen der Herstellungsbeispiel überlegene Wirksamkeit gegenüber dem Stand der Technik : 13, 4, 7, 5.

## Herstellungsbeispiele

### Beispiel 1

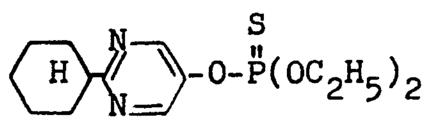

Ein Gemisch aus 300 ml Acetonitril, 17,8 g (0,1 Mol) 2-Cyclohexyl-5-hydroxy-pyrimidin, 20,7 g (0,15 Mol) Kaliumcarbonat und 18,8 g (0,1 Mol) O,O-Diäthylthiono-phosphorsäurediesterchlorid wird 2 Stunden bei 45 °C gerührt. Dann gießt man das Reaktionsgemisch in 400 ml Toluol und wäscht es zweimal mit je 300 ml Wasser. Die Toluollösung wird über Natriumsulfat getrocknet und im Vakuum eingedampft. Den Rückstand destilliert man im Hochvakuum an. Man erhält so 21,7 g (66 % der Theorie) O,O-Diäthyl-O-(2-cyclohexyl-pyrimidin(5)yl)-thiono-phosphorsäureester in Form eines braunen Öles mit dem Brechungsindex $n_D^{23}$ : 1,5158.

In analoger Weise können die folgenden Verbindungen der Formel

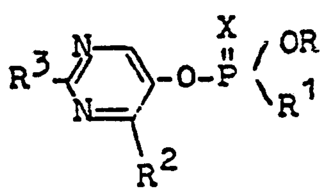

(I)

hergestellt werden :

| Beispiel Nr. | R | $R^1$ | $R^2$ | $R^3$ | X | Ausbeute (% der Theorie) | Physikal. Daten (Brechungsindex; Schmelzpunkt °C) |
|---|---|---|---|---|---|---|---|
| 2 | $C_2H_5$ | $NH-C_3H_7-iso$ | H | 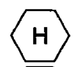 | S | 51 | $n_D^{23}$: 1,524 6 |
| 3 | $CH_3$ | $OCH_3$ | H | 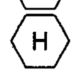 | S | 64 | $n_D^{23}$: 1,528 7 |
| 4 | $C_2H_5$ | $OC_2H_5$ | H | 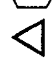 | S | 78 | $n_D^{24}$: 1,514 2 |

| Beispiel Nr. | R | $R^1$ | $R^2$ | $R^3$ | X | Ausbeute (% der Theorie) | Physikal. Daten (Brechungsindex; Schmelzpunkt °C) |
|---|---|---|---|---|---|---|---|
| 5 | $C_2H_5$ | $NH-C_3H_7-iso$ | H | 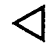 | S | 62 | 49 |
| 6 | $CH_3$ | $OCH_3$ | H | 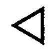 | S | 43 | $n_D^{24}$: 1,539 0 |
| 7 | $C_3H_7-n$ | $OC_2H_5$ | H | 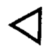 | S | 71 | $n_D^{25}$: 1,512 8 |
| 8 | $C_2H_5$ | $NH-C_2H_5$ | H | 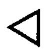 | S | 74 | $n_D^{26}$: 1,531 0 |
| 9 | $C_2H_5$ | $OC_2H_5$ | H | 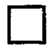 | S | | |
| 10 | $C_2H_5$ | $OC_2H_5$ | $CH_3$ | 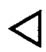 | S | | |
| 11 | $C_2H_5$ | $OC_2H_5$ | H | 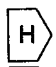 | S | 80 | $n_D^{23}$: 1,516 4 |
| 12 | $C_2H_5$ | $OC_3H_7-n$ | H | 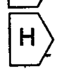 | S | | |
| 13 | $C_2H_5$ | $CH_3$ | H | 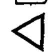 | S | 72 | $n_D^{25}$: 1,542 8 |
| 14 | $C_2H_5$ | $OC_2H_5$ | H | 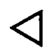 | O | | |
| 15 | $C_2H_5$ | $NH-C_3H_7-iso$ | H | 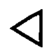 | O | | |
| 16 | $C_2H_5$ | 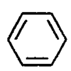 | H | 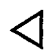 | S | 74 | $n_D^{25}$: 1,581 5 |
| 17 | $C_2H_5$ | $SC_3H_7-n$ | H | 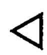 | S | | |
| 18 | $C_2H_5$ | 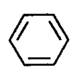 | H | 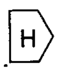 | S | | |
| 19 | $C_2H_5$ | $NH-C_2H_5$ | H | 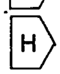 | S | 66 | $n_D^{23}$: 1,532 9 |
| 20 | $C_2H_5$ | $SC_3H_7-n$ | H | 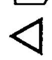 | O | | |
| 21 | $C_2H_5$ | $C_2H_5$ | H | 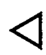 | S | | |
| 22 | $CH_3$ | $C_2H_5$ | H | 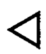 | S | | |
| 23 | $C_3H_7-iso$ | $CH_3$ | H | 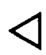 | S | 67 | $n_D^{26}$: 1,523 3 |
| 24 | $CH_3$ | $NH-C_3H_7-iso$ | H | 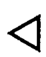 | S | | |
| 25 | $CH_3$ | $NH-CH_3$ | H | 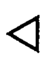 | S | 66 | $n_D^{26}$: 1,546 0 |
| 26 | $C_2H_5$ | $NH-CH_3$ | H | 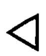 | S | | |
| 27 | $CH_3$ | $NH-C_2H_5$ | H | 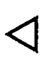 | S | | |
| 28 | $C_2H_5$ | $NH-C_3H_7-iso$ | H | 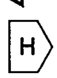 | S | 55 | $n_D^{23}$: 1,524 7 |

Die als Ausgangsmaterialien einzusetzenden 5-Hydroxypyrimidine können z.B. wie folgt hergestellt werden :

Beispiel a

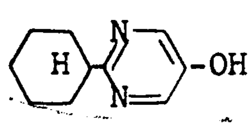

Zu einer Mischung aus 116,2 g (0,72 Mol) Cyclohexyl-amidin-Hydrochlorid, 150 ml Methanol und 0,8 Mol einer Lösung von Natriummethylat in Methanol gibt man bei Raumtemperatur 83,8 g (0,65 Mol) 2-Methoxy-3-dimethyl-aminoacrolein (Herstellung s.H. Plumpe u.E. Schegk, Archiv der Pharmazie *300* (1967) s. 704-708). Das Gemisch wird 5 Stunden unter Rückfluß gekocht, auf Raumtemperatur abgekühlt und vom ausgefallenen Salz abgesaugt. Man wäscht mit 100 ml Methanol nach, gibt zum Filtrat eine Lösung von 56 g (1 Mol) Kaliumhydroxyd in 100 ml Wasser und erhitzt dann die Lösung in einem Autoklaven 4 Stunden auf 190 °C. Dann destilliert man das Methanol im Vakuum ab, versetzt den Rückstand mit 50 ml Eiswasser und bringt die Lösung unter Kühlen durch Zugabe von konzentrierter Salzsäure auf einen pH-Wert von 4,5. Nach 30 Minuten saugt man das auskristallisierte Produkt ab und erhält so 96 g (83 % der Theorie) an 2-Cyclohexyl-5-hydroxy-pyrimidin als schwach braun gefärbtes Kristall-pulver mit dem Schmelzpunkt 163 °C.

Analog können die folgenden Verbindungen der Formel

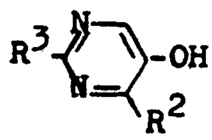

hergestellt werden :

| Beispiel | $R^2$ | $R^3$ | Ausbeute (% der Theorie) | Schmelzpunkt (°C) |
|---|---|---|---|---|
| b | H | 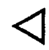 | 85 | 187 |
| c | H | 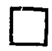 | | |
| d | H | 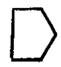 | 67 | 220 |
| e | $CH_3$ | 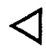 | | |

**Ansprüche**

1. 2-Cycloalkyl-pyrimidin(5)yl-(thiono) (thiol)-phosphor (phosphon)-säureester bzw. -esteramide der Formel I

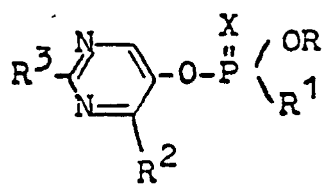

(I)

in welcher

R für Alkyl steht,
$R^1$ für Alkyl, Alkoxy, Alkylthio, Alkylamino oder Phenyl steht,
$R^2$ für Wasserstoff oder Alkyl steht,
$R^3$ für Cycloalkyl steht und
X für Sauerstoff oder Schwefel steht.

2. Verfahren zur Herstellung der 2-Cycloalkyl-pyrimidin(5)yl-(thiono) (thiol)-phosphor (phosphon)-säureester bzw. -esteramide der Formel II

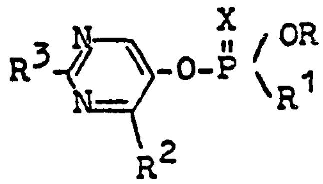

(I)

in welcher
R für Alkyl steht,
$R^1$ für Alkyl, Alkoxy, Alkylthio, Alkylamino oder Phenyl steht,
$R^2$ für Wasserstoff oder Alkyl steht,
$R^3$ für Cycloalkyl steht und
X für Sauerstoff oder Schwefel steht
dadurch gekennzeichnet, daß man (Thiono) (Thiol) Phosphor (phosphon)-säureesterhalogenide bzw. (Thiono)-phosphorsäureesteramidhalogenide der Formel II

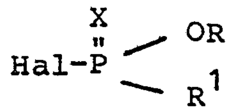 (II)

in welcher
R, $R^1$ und X die oben angegebene Bedeutung haben und
Hal für Chlor oder Brom steht, mit 5-Hydroxy-pyrimidinen der Formel III

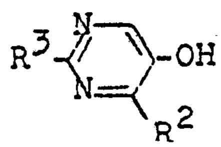 (III)

in welcher
$R^2$ und $R^3$ die oben angegebene Bedeutung haben, gegebenenfalls in Gegenwart eines Säureakzeptors und gegebenenfalls in Gegenwart eines inerten Verdünnungsmittels umsetzt.

3. Insektizide, akarizide und nematizide Mittel, gekennzeichnet durch einen Gehalt an mindestens einem 2-Cycloalkyl-pyrimidin(5)yl-(thiono) (thiol)-phosphor (phosphon)-säureester bzw. -esteramid der Formel (I)

4. Verwendung von 2-Cycloalkyl-pyrimidin(5)yl-(thiono) (thiol)-phosphor (phosphon)-säureestern bzw. -esteramiden der Formel (I) zur Bekämpfung von Insekten, Spinnentieren oder Nematoden.

5. Verfahren zur Herstellung insektizider, akarizider oder nematizider Mittel, dadurch gekennzeichnet, daß man 2-Cycloalkyl-pyrimidin(5)yl-(thiono) (thiol)-phosphor (phosphon)-säureester bzw. -esteramide der Formel (I) mit Streckmitteln und/oder oberflächenaktiven Mitteln vermischt.

**Claims**

1. 2-Cycloalkyl-pyrimidin-5-yl-(thiono) (thiol)-phosphoric (phosphonic) acid esters and ester-amides of the formula I

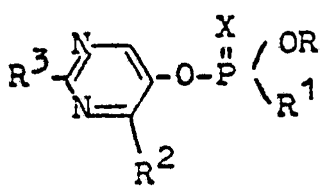 (I)

in which
R represents alkyl,
$R^1$ represents alkyl, alkoxy, alkylthio, alkylamino or phenyl,
$R^2$ represents hydrogen or alkyl,
$R^3$ represents cycloalkyl and
X represents oxygen or sulphur.

2. Process for the preparation of the 2-cycloalkyl-pyrimidin-5-yl-(thiono) (thiol)-phosphoric (phosphonic) acid esters or ester-amides of the formula I

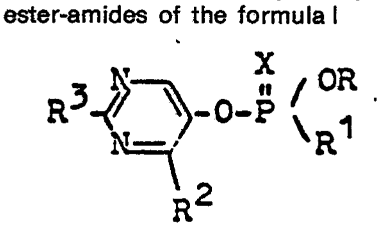 (I)

in which

R represents alkyl,

$R^1$ represents alkyl, alkoxy, alkylthio, alkylamino or phenyl,

$R^2$ represents hydrogen or alkyl,

$R^3$ represents cycloalkyl and

X represents oxygen or sulphur,

characterised in that (thiono) (thiol) phosphoric (phosphonic) acid ester halides or (thiono)-phosphoric acid ester amide halides of the formula II

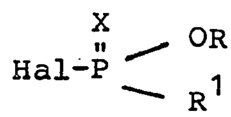

(II)

in which

R, $R^1$ and X have the meaning indicated above and

Hal represents chlorine or bromine, are reacted with 5-hydroxy-pyrimidines of the formula III

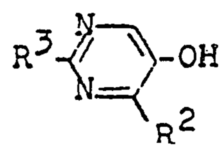

(III)

in which

$R^2$ and $R^3$ have the meaning indicated above, if appropriate in the presence of an acid acceptor and if appropriate in the presence of an inert diluent.

3. Insecticidal, acaricidal and nematicidal compositions characterised in that they contain at least one 2-cycloalkylpyrimidin-5-yl-(thiono) (thiol)-phosphoric (phosphonic) acid ester or ester-amide of the formula (I).

4. Use of 2-cycloalkyl-pyrimidin-5-yl-(thiono) (thiol)-phosporic (phosphonic) acid esters or ester-amides of the formula (I) for combating insects, arachnids or nematodes.

5. Process for the preparation of insecticidal, acaracidal or nematicidal compositions, characterised in that 2-cycloalkyl-pyrimidin-5-yl-(thiono) (thiol)-phosphoric (phosphonic) acid ester or ester-amides of the formula (I) are mixed with extenders and/or surface-active agents.

**Revendications**

1. Esters ou esteramides 2-cycloalcoylpyrimidin(5)yl-(thiono) (thiol)-phosphor (phosphon)iques de formule I

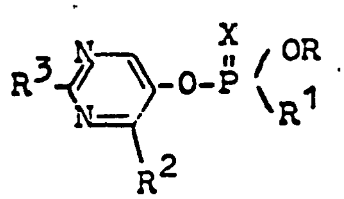

(I)

dans laquelle

R représente un alcoyle,

$R^1$ représente un alcoyle, alcoxy, alcoylthio, alcoylamino ou phényle,

$R^2$ représente de l'hydrogène ou un alcoyle,

$R^3$ représente un cycloalcoyle et

X représente de l'oxygène ou du soufre.

2. Procédé de fabrication d'esters ou esteramides 2-cycloalcoyl-pyrimidin(5)yl-(thiono)(thiol)-phosphor(phosphon)iques de formule I

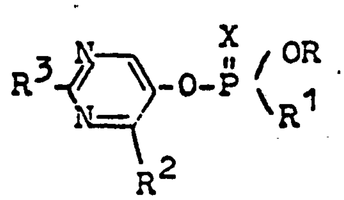

(I)

dans laquelle

R représente un alcoyle

$R^1$ représente un alcoyle, alcoxy, alcoylthio, alcoylamino ou phényle,

$R^2$ représente de l'hydrogène ou un alcoyle,

$R^3$ représente un cycloalcoyle et

X représente de l'oxygène ou du soufre,

caractérisé en ce qu'on fait réagir des halogénures d'esters (thiono) (thiol)-phosphor (phosphon)iques ou des halogénures d'esteramides (thiono)-phosphoriques de formule II

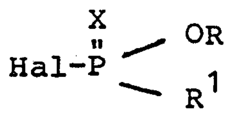

(II)

dans laquelle

R, $R^1$ et X ont la signification indiquée plus haut et

Hal représente du chlore ou du brome, avec des 5-hydroxy-pirimidines de formule III

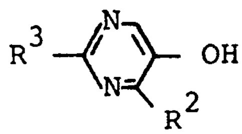

dans laquelle

$R^2$ et $R^3$ ont la signification indiquée plus haut, éventuellement en présence d'un accepteur d'acide et éventuellement en présence d'un diluant inerte.

3. Agents insecticides, acaricides et nématocides, caractérisés par une teneur en au moins un ester ou esteramide 2-cycloalcoyl-pyrimidine(5)yl-(thiono)(thiol)-phosphor (phosphon)ique de formule (I.).

4. Utilisation d'esters ou esteramides 2-cycloalcoyl-pyrimidin(5)yl-(thiono) (thiol) phosphor (phosphon)iques de formule (I) pour combattre les insectes, les acariens ou nématodes.

5. Procédé de fabrication d'agents insecticides, acaricides ou nématocides, caractérisé en ce qu'on mélange des esters ou esteramides 2-cycloalcoyl-pyrimidin(5)yl-(thiono) (thiol)-phosphor (phosphon)iques de formule (I) avec des diluants et/ou des agents tensioactifs.